# EUROPÄISCHE PATENTSCHRIFT

(11) **EP 0 880 535 B1**
(45) Veröffentlichungstag und Bekanntmachung des Hinweises auf die Patenterteilung: **17.09.2003**
(21) Anmeldenummer: 96923854.2
(22) Anmeldetag: 16.07.1996
(51) Int. Cl.: C07H 1/08, C12Q 1/68, B01F 11/00, B01F 13/04, C12N 15/10, C07H 21/00

(54) **VERFAHREN UND VORRICHTUNG ZUR SIMULTANEN ISOLIERUNG VON GENOMISCHER DNS UND HOCHREINER TOTAL RNS**
METHOD AND DEVICE FOR THE SIMULTANEOUS ISOLATION OF GENOMIC DNA AND HIGH-PURITY TOTAL RNA
PROCEDE ET DISPOSITIF POUR L'ISOLEMENT SIMULTANE DE L'ADN GENOMIQUE ET DE L'ARN TOTAL DE HAUTE PURETE

(30) Priorität: 31.01.1996 DE 29601618 U
(43) Veröffentlichungstag der Anmeldung: 02.12.1998
(73) Patentinhaber: Invitek Gesellschaft für Biotechnik & Biodesign mbH., 13125 Berlin (DE)
(72) Erfinder: HILLEBRAND, Timo, D-12619 Berlin (DE); BENDZKO, Peter, D-12623 Berlin (DE)
(74) Vertreter: Baumbach, Friedrich
(86) Internationale Anmeldenummer: DE9601291
(87) Internationale Veröffentlichungsnummer: WO97028171

(56) Entgegenhaltungen:
- WO-A-95/28409
- WO-A-95/34569
- DE-A- 4 422 044
- DE-A- 4 447 015

## Beschreibung

Die Erfindung betrifft ein Verfahren zur schnellen simultanen Isolierung von genomischer Desoxyribonukleinsäure (DNS) und zellulärer Total Ribonukleinsäure (RNS) aus unterschiedlichen Ausgangsmaterialien.
Es ist für eine Vielzahl von biologisch, molekularbiologisch, medizinisch-analytisch sowie biochemisch arbeitenden Laboratorien von großer Bedeutung. Damit sind Anwendungsgebiete der Erfindung die Molekularbiologie, Biochemie, Gentechnik, Medizin, Veterinärmedizin und alle angrenzenden Gebiete.
Die simultane Isolierung von genomischer DNS wie auch zellulärer Total RNS aus ein und demselben biologischen Ausgangsmaterial ist bis zum heutigem Zeitpunkt an nur einige wenige praktizierbare Methoden gebunden. So beschreiben Raha, S., Merante, F., Proteau, G. und Reed, J.K. (GATA, 1990, 7(7): 173-177) eine Methode zur Trennung genomischer DNS und zellulärer Total RNS über selektive Präzipitationsschritte unter Verwendung von Lithiumchlorid. Eine weitere Möglichkeit der simultanen Isolierung von DNS und RNS basiert auf Durchführung einer Ultrazentrifugation durch einen Cäsiumchloridgradienten zur Pelletierung der RNS und anschließender Dialyse der DNS aus der Guanidinium-Phase (Coombs, L.M., Pigott, D., Proctor, A., Eydmann, M., Denner, J. und Knowles, M.A.; Anal. Biochem. (1990); 188; 338-343). Ein solches Verfahren benötigt einen erheblichen zeitlichen (mindestens 48 Stunden) sowie apparativen Aufwand (Ultrazentrifugationsentechnik, spezielle Spezialrotoren).
Ein zur Zeit relativ häufig genutztes und auch kommerziell verfügbares Verfahren beruht auf der Verwendung eines Reagenz aus Guanidinthiocyanat und Phenol. Das biologische Material wird in diesem Reagenz homogenisiert, wobei sich die RNS nach Zugabe von Chloroform und durchzuführender Phasentrennung in der wäßrigen Phase befindet und aus dieser präzipitiert wird. Die zurückbleibende Interphase bzw. phenolische Phase enthält sowohl Proteine als auch genomische DNS. Durch Veränderung des pH Wertes und erneuter Phasentrennung soll die genomische DNS ebenfalls in die wäßrige Phase überführt werden und wird aus dieser wiederum präzipitiert. /Chomczynski, P., Biotechniques 1993, 15(3): 532-536/
Prinzipiell muß nach dem Stand der Technik davon ausgegangen werden, daß die isolierte zelluläre Total RNS mit genomischer DNS kontaminiert ist.
So enthält die mittels der von Chomcynski entwickelten und genutzten Reagenz erhaltene wäßrige Phase neben der RNS auch genomische DNS, welche dann ebenfalls aus dieser Phase präzipitiert wird und sich damit in der finalen RNS-Präparation als kontaminierender Bestandteil befindet. Gerade die Kontamination isolierter zellulärer RNS mit genomischer DNS stellt für eine Vielzahl von weiteren Verwendungen der RNS ein gravierendes Problem dar.
So ist z.B die Anwendung eines RNAse Protection Assays zwingend an eine DNS freie RNS gebunden. Ferner muß auch für eine Vielzahl von RT-PCR-Reaktionen die verwendete RNS frei von einer Kontamination mit genomischer RNS sein. So besteht z.B. bei Expressionsuntersuchungen von cDNS-Konstrukten in transgenen Organismen als auch bei Nachweisen der Expression intronloser Gene wie auch von noch unbekannten Gensequenzen keine Möglichkeit nachzuweisen, ob das resultierende PCR-Fragment aus der kontaminierenden DNS oder aus der RNS amplifiziert wurde. Sowohl aus der genomischen DNS wie auch aus einer RNS abgeleitete Amplifikate hätten dieselbe Länge.
Darüber hinaus sind auch eine Reihe weiterer molekularbiologischer Verfahren, wie z.B. DDRT-PCR oder zellfreie Proteinbiosynthesen in Form gekoppelter in-vitro Transkriptions/Translationssysteme, auf eine DNS freie RNS-Präparation angewiesen.
Dies zeigt die Bedeutung der Isolierung von genomischer DNSfreier Total RNS.

Bekannt ist auch die Isolierung von Nukleinsäuren (genomische DNA, sowie zelluläre Gesamt RNA) in getrennten Verfahren, wobei das Ausgangsmaterial lysiert wird und das Lysat mit einem mineralischen Träger (SiO₂) inkubiert wird (WO-A-9 534569). Das in WO-A-9 534569 beschriebene Verfahren ist jedoch nicht dazu geeignet, DNA und RNA simultan zu isolieren.

Ein weiteres Problem beteht in der Präparationsdauer zur simultanen Isolierung von genomischer DNS und zellulärer Total RNS sowie in deren arbeitstechnischem Aufwand.
Das einzige zur Zeit kommerziell verfügbare Isolierungssystem benötigt für die Realisierung der simultanen Isolierung beider Nukleinsäurefraktionen mindestens 3 Stunden und ist mit einem nicht unerheblichen Aufwand an notwendigen Reaktionsgefäßen und Feinchemikalien belastet. Weiterhin benötigen alle diese Methoden auch eine relativ große Menge an biologischen Ausgangsmaterialien, so daß bei Vorhandensein von nur limitierter Mengen an Untersuchungsmaterialien meistens eine simultane Isolierung beider Nukleinsäuren nicht mehr möglich ist.

Die Erfindung hat deshalb das Ziel, eine simultane Isolierung von genomischer DNS und zellulärer Total RNS hoher Reinheit und ohne eine Kontamination mit genomischer DNS auch aus sehr kleinen Mengen unterschiedlicher Ausgangsmaterialien zu erreichen. Dabei soll das Verfahren sehr einfach handhabbar sein, nur geringe apparative Ausrüstung benötigen und gestatten, beide Nukleinsäurefraktionen sehr schnell zu isolieren.

Die Erfindung wird gemäß den Ansprüchen realisiert, die Unteransprüche sind Vorzugsvarianten.

Das Verfahren zur simultanen Isolierung von genomischer DNS und zellulärer Total RNS ist dadurch gekennzeichnet, daß die Nukleinsäuren enthaltenden Materialien lysiert, und das Lysat mit einem mineralischen Träger oder anderen DNS-bindenden Materialien inkubiert wird. Anschließend erfolgt a) die Abtrennung des Trägers vom Lysat durch Zentrifugation, Zugabe von Phenol, Chloroform und Natriumacetat zum Lysat und Präzipitation der Total RNS nach Phasentrennung aus der wäßrigen Phase durch Zugabe von Isopropanol und b) Waschen des Trägers mit einem Waschpuffer und Ablösung der trägerfixierten genomischen DNS vom Trägermaterial mit einem Puffer geringer Salzkonzentration.

Die im erfindungsgemäßen Verfahren erhaltene Total RNS ist undegradiert und von exzellenter Qualität (OD₂₆₀:OD₂₈₀= 1.8-2.0). Von entscheidener Bedeutung ist dabei, daß keine Kontamination genomischer DNS mehr vorliegt. Dies bedeutet einen erheblichen Vorteil gegenüber den meisten bisher Verwendung findenden Präperationsmethoden. Auch die ebenfalls aus ein und derselben biologischen Probe isolierte genomische DNS ist von exzellenter Qualität und für eine Vielahl weiterer Verfahren als Substrat verwendbar. Das erfindungsgemäße Verfahren zeichnet sich weiterhin durch seine Einfachheit aus, benötigt nur geringe Mengen an Feinchemikalien wie auch Zentrifugengefäße und minimiert auch die Menge und notwendige Zeitdauer des Umganges mit toxischen organischen Solventien (benötigt nur einen Phenol/Chloroform Extraktionsschritt). Das Verfahren gestattet es sowohl genomische DNS als auch zelluläre Total RNS innerhalb von weniger als 1,5 Stunden zu isolieren. Dies bedeutet eine drastische Reduzierung der Präparationsdauer im Vergleich zu allen z.Z. bekannten diesbezüglichen Verfahren. Die Bindung der Desoxyribonukleinsäure erfolgt an der Oberfläche von hochdispersen und nichtporösen Feststoffpartikeln, vorzugsweise an hochdisperse, nichtporöse SiO₂- Partikeln mit einer Korngröße von 7 bis 300 nm und einer spezifischen Oberfläche von 10 bis 300 m²/g, besonders bevorzugt mit einem Partikeldurchmesser von 40 nm bei einer aktiven Oberfläche von ca. 50 m²/g. Die Bindung der Desoxyribonukleinsäure an das verwendete Trägermaterial wird durch chaotrope Salze des Lysepuffers vermittelt. Die Lyse der Ausgangsmaterialien und die Bindung an das Trägermaterial erfolgen im selben Reaktionsgefäß.

Gegebenenfalls werden zur Lyse des die Nukleinsäuren enthaltenden Ausgangsmaterialien chaotrope Salze wie zum Beispiel Guanidinthiocyanat, Guanidinhydrochlorid, Litiumchlorid oder Litiumchlorid/Harnstoff-Gemische mit Ionenstärken >4M eingesetzt.

Vorzugsweise wird der Träger mit der fixierten genomische DNS vom Lysat durch einen kurzen Zentrifugationsschritt abgetrennt.

Die am Träger gebundene genomische DNS wird besonders bevorzugt mit einem Waschpuffer, vorzugsweise bestehend aus 50 mM NaCl, 10 mM Tris HCl und 1 mM EDTA sowie 70%v/v Ethanol, gewaschen und mit einem Puffer niedriger Salzkonzentration (10 mM Tris HCl, 1 mM EDTA) bei einer Temperatur von 48-56°C, vorzugsweise 52°C, eluiert.

Das Verfahren wird als batch- Verfahren oder als chromatographisches Verfahren durchgeführt.

Das sehr einfache und nur wenige experimentelle Schritte umfassende Verfahren ist in idealer Weise für eine breite Anwendung in medizinischen Diagnostik-Laboratorien geeignet und steht in diesem Zusammenhang auch Anwendern zur Verfügung, welche nicht über spezielle molekularbiologisch-biochemische Kenntnisse verfügen.

Mit dem erfindungsgemäßen Verfahren ist unter anderem die Voraussetzung gegeben, aus limitierten Mengen an Untersuchungsmaterialien sowohl die DNS als auch die RNS zu isolieren. So ist es möglich, genomische DNS und zelluläre Total RNS sogar aus sehr kleinen Mengen (< 10⁵ Zellen; < 1 mg Gewebematerial) zu isolieren. Dies erlaubt Untersuchungen von Genen (DNS-Untersuchung) und deren Expression (RNS-Untersuchung). Vor allem quantitative Abnormitäten von Genen und deren RNS-Expression scheinen eine wesentliche Bedeutung von Vorgängen während der Kanzerogenese und Metastasierung wie auch bei der postoperativen Progression von Tumorpatienten zu spielen. Die Möglichkeit des Findens korrelativer Zusammenhänge bezüglich der Anzahl bestimmter tumorassoziierter DNS-Sequenzen, deren Struktur (Sequenzinformation) und deren Expression (RNS) ist somit von entscheidender Bedeutung für ein besseres Verstehen von Zusammenhängen pathogener Mechanismen. Ferner erlaubt eine Methode der simultanen Isolierung von DNS und Total RNS auch die Untersuchung unterschiedlicher Spleißing-Mechanismen (z.B. Alternatives Spleißen, Trans-Spleißen). Die Untersuchung von Spleißvorgängen hat sowohl im Bereich der Grundlagenforschung (Untersuchung von Vorgängen der Genregulation) wie auch auf medizinischen Gebiet (Aufklärung immunologischer Phänomene bei parasitären Erkrankungen; z.B. nach Infektion mit Afrikanischen Trypanospomen) ebenfalls eine große Bedeutung.
Die Mehrzahl solcher Studien scheitert am Fehlen eines geeigneten methodischen Instrumentariums zur simultanen Isolierung von DNS und RNS, vor allem bei Vorhandensein nur geringer Mengen an Untersuchungsmaterialien.

Mit den erfindungsgemäßen Verfahren besteht die Möglichkeit der simultanen Isolierung von genomischer DNS und zellulärer Total RNS aus bakteriellen Lysaten, aus Zellkulturen, intakten oder gefrorenen Gewebeproben, Spermien, Körperflüssigkeiten, Pflanzenzellen, Hefezellen sowie Blutserum, Blutplasma und Vollblut.
Die erfindungsgemäßen Verfahrensvarianten erlauben die simultane Isolierung beider Nukleinsäuren (DNS und RNS) mit einem extrem geringen zeitlichen sowie apparativen Aufwand.

Es ist keine zeitaufwendige Proteinase-Verdauung notwendig. Der geringe zeitliche Aufwand der simultanen Isolierung von DNS und RNS aus ein und demselben Ausgangsmaterial stellt für eine Vielzahl von potentiellen Anwendern eine enorm wichtige Größe dar und bedeutet damit einen entscheidenden Vorteil gegenüber anderen Verfahren. Die Eigenschaft des verwendeten Lysepuffers, sowohl die zelluläre Integrität zu zerstören wie auch endogene und exogene DNAsen und vor allem RNAsen hochpotent zu inaktivieren, gestattet es darüber hinaus, DNS und RNS aus Frischpräparaten unter Feldbedingungen ( z.B. bei Expeditionen, nach Operationen) zu isolieren, ohne zusätzliche Kühlbedingungen unter Lysepuffer zu lagern und zu transportieren und die Ribonukleinsäuren ohne Verlust ihrer biologischen Aktivität einer weiteren Verwendung zuzuführen.

Die Erfindung soll nachfolgend an Ausführungsbeispielen näher erläutert werden.

### Beispiel 1

Simultane Isolierung genomischer DNS und zellulärer Total RNS aus einer eukaryontischen Monolayer-Zellkultur (25 cm² Flasche ; ca 5x10⁶ Zellen)

Ernten der Zellen mit einem Scrapper und Überführen der geernteten Zellen in 1.5 oder 2.0 ml Eppendorf-Reaktionsgefäß. Lyse der Zellen durch Zugabe von Lysepuffer (Guanidinthiocyanat; N-Lauryl-Sarcosyl; DTT; Natriumcitrat: )und Plazieren von bis zu 12 Reaktionsgefäße in die Vorrichtung. Zugabe des Trägermaterials zur Zell-Lyse-Suspension, kurzes Vortexen, Inkubation für 5 Minuten in einem Eisbad und anschließende Pelletierung des Trägermaterials durch kurze Zentrifugation in einer Tischzentrifuge (30 Sekunden).

Überführung des Überstandes in ein neues Eppendorf-Zentrifugengefäß und Zugabe von Phenol (wassergesättigt oder Tris-gepuffert), Chloroform und Natriumacetat und 5 minütige Inkubation auf Eis. Nach erfolgter Phasentrennung durch Zentrifugation wird die obere wäßrige Phase in ein neues Eppendorf-Zentrifugengefäß überführt, mit einem gleichem Volumen Isopropanol versetzt und zur Präzipitation der RNS für 20-30 Minuten bei -20°C inkubiert. Die am Trägerpellet gebundene genomische DNS wird während der Isopropanolfällung der RNS mit Waschpuffer (50 mM NaCl; 10 mM Tris HCl; 1 mM EDTA; 70% v/v Ethanol) versetzt und gewaschen. Das Waschen der trägerfixierten genomischen DNS erfolgt dabei mittels der Vorrichtung, welche die Resuspendierung des Trägermaterials realisiert. Anschließend wird die genomischen DNS durch Zugabe eines Elutionspuffers (Tris; EDTA) bei 52°C vom Trägermaterial abgelöst, der Träger durch kurze Zentrifugation von der eluierten genomische DNS abgetrennt und diese in ein neues Reaktionsgefäß überführt.
Das nach Inkubation bei -20°C und anschließender Zentrifugation erhaltene RNS-Pellet wird zweimal mit 70%igem Ethanol gewaschen und nach erfolgter vollständiger Entfernung des Ethanols das Pellet in RNAse freiem TE Puffer oder DEPCbehandeltem Aqua Bidest (Diethylpyrocarbonat) aufgenommen.

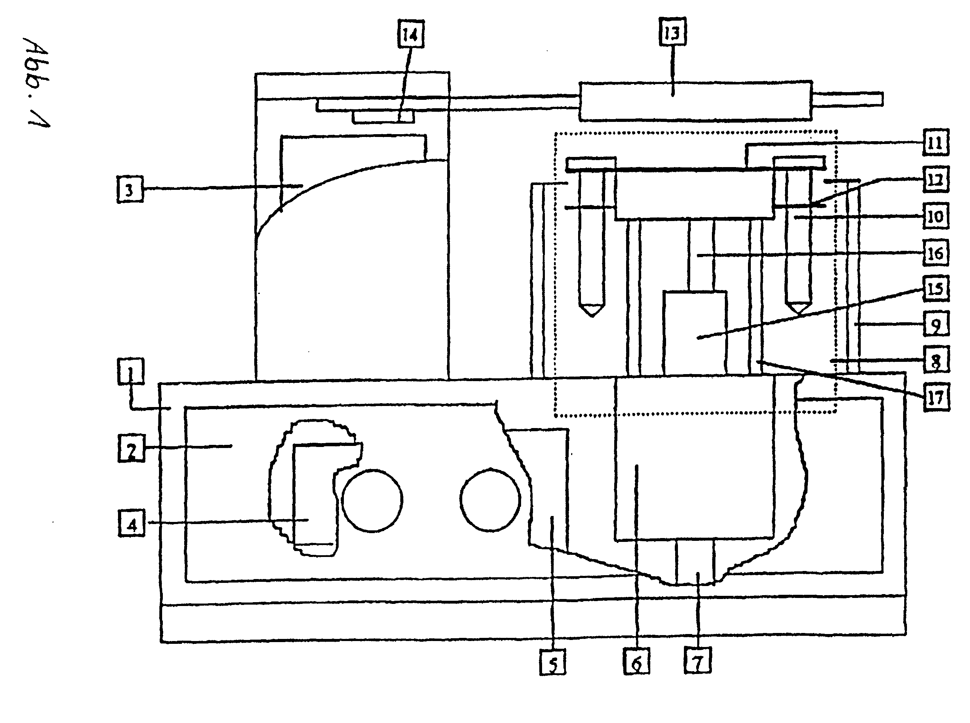

## Patentansprüche

1. Verfahren zur simultanen Isolierung von genomischer DNS und zellulärer Total RNS, **gekennzeichnet durch** Lyse von diese Nukleinsäuren enthaltenden Materialien, Inkubation des Lysates mit einem mineralischen Träger oder anderen DNS-bindenden Materialien, sowie
a) Abtrennung des Trägers vom Lysat **durch** Zentrifugation, Zugabe von Phenol, Chloroform und Natriumacetat zum Lysat und Präzipitation der Total RNS nach Phasentrennung aus der wäßrigen Phase **durch** Zugabe von Isopropanol und
b) Waschen des Trägers mit einem Waschpuffer und Ablösung der trägerfixierten genomischen DNS vom Trägermaterial mit einem Puffer geringer Salzkonzentration.

2. Verfahren nach Anspruch 1, **dadurch gekennzeichnet, daß** zur Lyse des Ausgangsmaterials chaotrope Salze, vorzugsweise Guanidinthiocyanat, Guanidinhydrochlorid, Litiumchlorid oder Litiumchlorid/Harnstoff-Gemische mit Ionenstärken >4M eingesetzt werden.

3. Verfahren nach Anspruch 1 oder 2 **dadurch gekennzeichnet, daß** als Trägermaterialien zur Bindung der genomischen DNS hochdisperse, nichtporöse SiO₂- Partikeln mit einer Korngröße von 7-300 nm, vorzugsweise 40 nm, bei einer spezifischen Oberfläche von 10-300 m²/g, vorzugsweise 50 m²/g, eingesetzt werden.

4. Verfahren nach Anspruch 1 -3, **dadurch gekennzeichnet**, das der Träger mit fixierter genomischer DNS vom Lysat durch einen kurzen Zentrifugationsschritt abgetrennt wird.

5. Verfahren nach Anspruch 1-4, **dadurch gekennzeichnet, daß** am Träger gebundene genomische DNS mit einem Waschpuffer, vorzugsweise bestehend aus 50 mM NaCl, 10 mM Tris HCl und 1 mM EDTA sowie 70%v/v Ethanol, gewaschen wird.

6. Verfahren nach Anspruch 1-5, **dadurch gekennzeichnet, daß** trägerfixierte genomische DNS mit einem Puffer niedriger Salzkonzentration (10 mM Tris HCl, 1 mM EDTA) bei einer Temperatur von 48-56°C, vorzugsweise 52°C, eluiert wird.

7. Verfahren nach Anspruch 1-6, **dadurch gekennzeichnet, daß** es als batch- Verfahren durchgeführt wird.

8. Verfahren nach Anspruch 1-6, **dadurch gekennzeichnet, daß** es als chromatographisches Verfahren durchgeführt wird.

## Claims

1. Method for the simultaneous isolation of genomic DNA and cellular total RNA, wherein there is a lysis of materials containing said nucleic acids, incubation of the lysate with a mineral carrier or other DNA-binding materials, as well as
a) separation of the carrier from the lysate by means of centrifugation, addition of phenol, chloroform and sodium acetate to the lysate and precipitation of the total RNA following phase separation from the watery phase by addition of isopropyl alcohol and
b) washing of the carrier with a washing buffer and separation of the carrier-fixed genomic DNA from the carrier material with a buffer of low saline concentration.

2. Method according to Claim 1, wherein chaotropic salts, preferably guanidine thiocyanate, guanidine hydrochloride, lithium chloride or lithium chloride/urea mixtures with ion sizes > 4 M are used for the lysis of the initial material.

3. Method according to Claim 1 or 2, wherein highly dispersive, non-porous SiO₂ particles with a grain size of 7 - 300 nm, preferably 40 nm, with a specific surface area of 10-300 m²/g, preferably 50 m²/g are used as carrier materials for the binding of the genomic DNA.

4. Method according to Claims 1 to 3, wherein the carrier with the fixed genomic DNA is separated from the lysate by means of a brief centrifugation step.

5. Method according to Claims 1 to 4, wherein genomic DNA bound to the carrier is washed with a washing buffer, preferably comprising 50 mM NaCl, 10 mM Tris HCl and 1 mM EDTA as well as 70% v/v ethanol.

6. Method according to Claims 1 to 5, wherein carrier-fixed genomic DNA is eluted with a buffer of low saline concentration (10 mM Tris HCl, 1 mM EDTA) at a temperature of 48-56°C, preferably 52°C.

7. Method according to Claims 1 to 6, wherein it is carried out as a batch method.

8. Method according to Claims 1 to 7, wherein it is carried out as a chromatographic method.

## Revendications

1. Procédé permettant l'isolation simultanée d'A.D.N. génomique et d'A.R.N. Total cellulaire, **se caractérisant par** la lyse de matériaux contenant ces acides nucléiques, par l'incubation du lysat avec un support minéral ou d'autres matériaux liant l'A.D.N. ainsi que par
a) la séparation du support du lysat par centrifugation, addition de phénol, de chloroforme et d'acétate de sodium au lysat et précipitation de l'A.R.N. Total après séparation des phases à partir de la phase aqueuse par addition d'alcool isopropylique et
b) le lavage du support avec un tampon de lavage et séparation de l'A.D.N. génomique fixé sur le support du matériau support avec un tampon dont la concentration en sel est faible.

2. Procédé selon la revendication 1, **se caractérisant par le fait que** des sels chaotropes, de préférence du thiocyanate de guanidine, de l'hydrochlorure de guanidine, du chlorure de lithium ou du chlorure de lithium/mélanges d'urée avec forces ioniques >4M seront employés pour effectuer la lyse du matériau de départ.

3. Procédé selon la revendication 1 ou 2, **se caractérisant par le fait que** des particules SiO₂ très finement divisées, non poreuses avec une grosseur de grain de 7-300 nm, de préférence 40 nm, pour une surface spécifique de 10-300 m²/g, de préférence 50 m²/g seront employées en tant que matériaux supports pour assurer la liaison de l'A.D.N. génomique.

4. Procédé selon la revendication 1 - 3, **se caractérisant par le fait que** le support avec l'A.D.N. génomique fixé sera séparé du lysat par une étape de centrifugation.

5. Procédé selon la revendication 1 - 4, **se caractérisant par le fait que** l'A.D.N. génomique lié au support sera lavé par un tampon de lavage, se composant de préférence de 50 mM NaCl, 10 mM Tris HCL et 1 mM EDTA ainsi que de 70% v/v éthanol.

6. Procédé selon la revendication 1 - 5, **se caractérisant par le fait que** l'A.D.N. génomique fixé au support sera élué par un tampon dont la concentration en sel est faible (10 mM Tris HCl, 1 mM EDTA) à une température de 48-56 °C, de préférence 52 °C.

7. Procédé selon la revendication 1 - 6, **se caractérisant par le fait que** ce procédé est exécuté en tant que procédé batch.

8. Procédé selon la revendication 1 - 6, **se caractérisant par le fait que** ce procédé est exécuté en tant que procédé de chromatographie.
